# EUROPÄISCHE PATENTSCHRIFT

(11) **EP 1 231 198 B1**
(45) Veröffentlichungstag und Bekanntmachung des Hinweises auf die Patenterteilung: **12.10.2005**
(21) Anmeldenummer: 02003057.3
(22) Anmeldetag: 12.02.2002
(51) Int. Cl.: C07C 45/50, B01J 10/00, B01J 19/24

(54) **Hydroformylierung**
Hydroformylation
Hydroformylation

(30) Priorität: 13.02.2001 DE 10106482
(43) Veröffentlichungstag der Anmeldung: 14.08.2002
(73) Patentinhaber: BASF Aktiengesellschaft, 67056 Ludwigshafen (DE)
(72) Erfinder: Zehner, Peter, 67071 Ludwigshafen (DE); Nilles, Michael, 67240 Bobenheim-Roxheim (DE)
(74) Vertreter: Kinzebach, Werner

(56) Entgegenhaltungen:
- EP-A- 1 057 524
- DE-A- 3 220 858
- DE-C- 926 846
- US-A- 3 929 900

## Beschreibung

Die Erfindung betrifft ein Verfahren zur Hydroformylierung von Olefinen mit wenigstens 6 Kohlenstoffatomen und eine Vorrichtung zur Durchführung des Verfahrens.

Die Hydroformylierung oder Oxo-Synthese ist ein wichtiges großtechnisches Verfahren und dient der Herstellung von Aldehyden aus Olefinen, Kohlenmonoxid und Wasserstoff. Diese Aldehyde können gegebenenfalls im gleichen Arbeitsgang oder nachfolgend in einem getrennten Hydrierschritt mit Wasserstoff zu den entsprechenden Alkoholen hydriert werden. Die Hydroformylierung erfolgt in Anwesenheit von homogen im Reaktionsmedium gelösten Katalysatoren. Als Katalysatoren werden dabei im Allgemeinen die Carbonylkomplexe von Metallen der VIII. Nebengruppe, insbesondere Co, Rh, Ir, Pd, Pt oder Ru eingesetzt, die unmodifiziert oder z. B. mit amin- oder phosphinhaltigen Liganden modifiziert sein können. Eine zusammenfassende Darstellung der großtechnisch ausgeübten Verfahren findet sich bei J. Falbe, "New Syntheses with Carbon Monoxide", Springer Verlag 1980, S. 162 ff.

Während kurzkettige Olefine vergleichsweise leicht hydroformyliert werden, bereitet die Hydroformylierung höherer Olefine mitunter Probleme. Dies liegt an der mit steigender Kettenlänge und zunehmenden Anteil an inneren, nicht endständigen Doppelbindungen abnehmenden Reaktionsgeschwindigkeit und außerdem an der Zunahme der Viskosität der Edukte und des Reaktionsgemisches. Zur Erzielung angemessener Umsätze sind daher lange Verweilzeiten notwendig, die jedoch infolge unerwünschter Nebenreaktionen, insbesondere der Bildung hochsiedender Reaktionsnebenprodukte, die Selektivität verschlechtern. Andererseits sind die Reinheitsanforderungen an das unmittelbare Verfahrensprodukt sehr hoch, weil dieses aufgrund seiner hohen Viskosität praktisch nicht auftrennbar ist und somit im Wesentlichen ohne weitere Reinigung eingesetzt werden muss. Die Verringerung des Reaktionsvolumens ist für die Wirtschaftlichkeit des Verfahrens von besonderer Bedeutung, da große Reaktionsvolumina bei den für die Verfahrensdurchführung vorgegebenen hohen Druckwerten sehr teuer sind.

Um das Reaktionsvolumen möglichst klein zu halten bzw. zur Erhöhung der Raumzeitausbeute wurde daher vorgeschlagen, anstelle eines total rückvermischten Reaktors (continuously stirred tank reactor, CSTR) eine Reaktorkaskadierung einzusetzen. Eine Kaskadierung kann durch verschiedene Maßnahmen erreicht werden, die jedoch jeweils die im Folgenden aufgeführten Nachteile aufweisen:
Eine gängige Methode ist die Kaskadierung durch Hintereinanderschalten von Reaktoren. Diese Methode ist jedoch die aufwendigste, da der Investitionsaufwand für die einzelnen Reaktoren, die dazugehörigen Verbindungen und der erforderliche Mess- und Regelaufwand mit der Anzahl der Reaktoren steigt.

Die WO 97/20793 beschreibt ein Verfahren zur Hydroformylierung in einem einzigen Reaktor, wobei eine Kaskadierung durch horizontal eingebaute Lochbleche vorgesehen ist. Die Rührorgane des zentral angeordneten Rührers sind in ihrer Ausbildung an die Öffnungen und Zwischenräume der Lochbleche angepasst. Ein solcherart ausgebildeter Reaktor hat insbesondere die folgenden Nachteile: Hochdruckapparate sind in der Regel als Zylinder ausgebildet, deren Enden geringere Durchmesser als der Mittelteil aufweisen, das heißt als sogenannte Doppelflaschenhalsapparate. Es ist apparativ sehr aufwendig, horizontale Lochbleche, deren Durchmesser dem Mittelteil entsprechen, durch derartige Flaschenhälse zu montieren. Die Kaskadierung mit horizontalen Lochblechen hat den weiteren Nachteil, dass diese Lochbleche im Falle einer Notentspannung einem hohen Druck standhalten und daher mit sehr großen Wandstärken dimensioniert werden müssen. Außerdem muss die in den Reaktionsraum eingeführte Rührerwelle gegen den hohen Druck abgedichtet werden.

Demgegenüber ist es Aufgabe der Erfindung, ein Verfahren zur Hydroformylierung von Olefinen mit wenigstens 6 Kohlenstoffatomen zur Verfügung zu stellen, das sich durch einen möglichst hohen Umsatz bei gegebenem Reaktorvolumen auszeichnet. Eine weitere Aufgabe ist die Bereitstellung einer Vorrichtung zur Durchführung des Verfahrens mit niedrigen Investitions- und Betriebskosten.

Erfindungsgemäß wird die Aufgabe gelöst durch ein kontinuierliches Verfahren zur Hydroformylierung von Olefinen mit wenigstens 6 Kohlenstoffatomen unter homogener Katalyse, bei dem man
a) einen vertikalen hochzylindrischen Reaktor (1) verwendet, dessen Innenraum mittels Einbauten (2) in wenigstens zwei Reaktionsräume unterteilt ist, die zumindest einen ersten und einen letzten Reaktionsraum umfassen und die sich im Wesentlichen in Reaktorlängsrichtung erstrecken und deren Länge mehr als 50 % der Reaktorlänge beträgt,
b) wenigstens ein Olefin zusammen mit Synthesegas am unteren Ende des ersten Reaktionsraums in den Reaktor einführt,
c) vom oberen Ende eines Reaktionsraumes ohne Rückvermischung mit dem Inhalt des Reaktionsraums ein teilumgesetztes Reaktionsgemisch zum unteren Ende eines nächsten Reaktionsraums führt; und
d) am oberen Ende des letzten Reaktionsraums das hydroformylierte Olefin entnimmt.

Die Erfindung betrifft auch einen zur Durchführung des vorstehenden Verfahrens geeigneten vertikalen hochzylindrischen Reaktor nach Anspruch 6.

Nach dem erfindungsgemäßen Verfahren lassen sich Olefine mit wenigstens 6, z. B. mit 9 bis 20, Kohlenstoffatomen hydroformylieren. Aus praktischen Gründen ist die Kettenlänge nach oben in der Regel bei etwa 700 Kohlenstoffatomen begrenzt. Das erfindungsgemäße Verfahren ist insbesondere für die Hydroformylierung von isomeren Olefingemischen geeignet, die durch Oligomerisierung von niederen Olefinen, wie Propen und Butenen, hergestellt werden. Zu den typischen Oligomerisaten, die als Ausgangsprodukte für das vorliegende Verfahren geeignet sind, zählen unter anderem Dimer-, Trimer- und Tetramerpropen, Dimer-, Trimer- und Tetramerbuten sowie Mischoligomerisate von Propenen und Butenen. Die Oligomerisate von Butenen sind nach bekannten Oligomerisierungsverfahren, z. B. nach dem Octol®-Prozess von Hüls und dem Dimersol®-Verfahren von IFP, großtechnisch zugänglich. Nach dem erfindungsgemäßen Verfahren können ferner lineare langkettige Olefine mit endständiger Doppelbindung, die z. B. nach dem SHOP®-Prozess oder Ziegler-Verfahren erhältlich sind, oder lineare langkettige Olefine mit innenliegender Doppelbindung hydroformyliert werden.

Weitere bevorzugte Einsatzolefine sind im Wesentlichen einfach ungesättigte Polyalkylene mit 30 bis 700 Kohlenstoffatomen, wie insbesondere Polybuten oder Polyisobuten.

Das erfindungsgemäße Verfahren findet unter homogener Katalyse statt. In der Regel wird hierzu mit dem Olefin und dem Synthesegas ein geeigneter Katalysator oder Katalysatorvorläufer in den Reaktor eingeführt. Bezüglich der eingesetzten Katalysatoren bzw. Katalysatorvorläufer gibt es keine wesentlichen Einschränkungen. In an sich bekannter Weise werden insbesondere Kobaltkatalysatoren, vorzugsweise Kobaltcarbonyle oder Kobaltcarbonylwasserstoff bzw. deren Vorläufer, wie insbesondere Kobalt(II)-salze, z. B. Kobalt(II)-formiat, Kobalt(II)-acetat oder Kobalt(II)-ethylhexanoat, eingesetzt.

Zweckmäßigerweise wird der Katalysator in gelöster Form im Einsatzolefin oder einem gegebenenfalls mitverwendeten organischen Lösungsmittel eingeführt. Hierzu kann man außerhalb des Reaktors eine wässrige Kobalt(II)-salzlösung mit Synthesegas in Kontakt bringen, wobei ein hydroformylierungsaktiver Kobaltkatalysator gebildet wird, und die den Kobaltkatalysator enthaltende wässrige Lösung gleichzeitig oder anschließend mit dem Einsatzolefin und/oder dem organischen Lösungsmittel in Kontakt bringen, wobei der Kobaltkatalysator in die organische Phase extrahiert wird.

Die Vorab-Bildung des Katalysators erfolgt vorzugsweise bei Temperaturen von 50 bis 200 °C, insbesondere 100 bis 160 °C und unter Drucken von 100 bis 400 bar, insbesondere 200 bis 300 bar. Es eignen sich übliche Apparate für Gas-flüssig-Reaktionen, wie Rührbehälter mit Begasungsrührer, Blasensäulen oder Rieselbettkolonnen. Mit Vorteil erfolgt die Vorcarbonylierung in Gegenwart von Aktivkohle, Zeolithen oder basischen Ionenaustauschen, die mit Kobaltcarbonyl beladen sind, gemäß Beschreibung in der DE-OS 2139630. Aus der so erhaltenen, Kobalt(II)-salze und Kobaltkatalysator enthaltenden wässrigen Lösung wird dann der Kobaltkatalysator in die zu hydroformylierenden Olefinen und/oder das gegebenenfalls mitverwendete organische Lösungsmittel extrahiert.

In vielen Fällen ist es im Hinblick auf den geringeren verfahrenstechnischen Aufwand bevorzugt, dass die Bildung des Kobaltkatalysators, die Extraktion des Kobaltkatalysators in die organische Phase und die Hydroformylierung der Olefine in einem Schritt erfolgen, indem die wässrige Kobalt(II)-salzlösung, die Olefine, Synthesegas und gegebenenfalls das organische Lösungsmittel gemeinsam in den Reaktor eingeführt werden. Die Ausgangsstoffe werden dabei so in die Reaktionszone eingebracht, dass eine gute Phasenvermischung erfolgt und eine möglichst hohe Phasenaustauschfläche erzeugt wird. Für die Dosierung können die dem Fachmann bekannten Dosiervorrichtungen, wie z. B. mit Füllkörpern befüllte Turbulenzrohre oder Mischdüsen für Mehrphasensysteme eingesetzt werden.

Zur Entfernung des Kobaltkatalysators wird der Reaktionsaustrag geeigneterweise nach Verlassen der Reaktionszone auf Mitteldruck, in der Regel 10 bis 30 bar, entspannt und in eine sogenannte Entkobaltungsstufe geleitet. In der Entkobaltungsstufe wird der Reaktionsaustrag in Gegenwart von wässriger, schwach saurer Kobalt(II)-salzlösung mit Luft oder Sauerstoff bei Temperaturen von vorzugsweise 90 bis 130 °C von Kobaltcarbonylkomplexen befreit. Die Entkobaltung kann gewünschtenfalls in einem mit Füllkörpern, wie z. B. Raschig-Ringen, befüllten Druckbehälter durchgeführt werden, in dem eine möglichst hohe Phasenaustauschfläche erzeugt wird. In einem nachgeschalteten Phasentrenngefäß wird die organische Produktphase von der wässrigen Phase getrennt. Bei der Entkobaltung wird der hydroformylierungsaktive Kobaltkatalysator unter Bildung von Kobalt(II)-salzen, überwiegend Kobalt(II)-formiat, zersetzt. Die wässrige Kobalt(II)-salzlösung wird zweckmäßigerweise in die Reaktionszone bzw. Katalysatorbildungsstufe zurückgeführt.

Alternativ können Rhodiumkatalysatoren, die durch stickstoff- oder phosphorhaltige Liganden modifiziert sein können, eingesetzt werden. Die Abtrennung des Rhodiumkatalysators vom Hydroformylierungsprodukt erfolgt im Allgemeinen durch Destillation, wobei der Rhodiumkatalysator zusammen mit hochsiedenden Bestandteilen als Rückstand verbleibt.

Synthesegas ist ein großtechnisch zugängliches Gemisch von Kohlenmonoxid und Wasserstoff. Die Zusammensetzung des im erfindungsgemäßen Verfahren eingesetzten Synthesegases kann in weiten Bereichen variieren. Das molare Verhältnis von Kohlenmonoxid und Wasserstoff beträgt in der Regel etwa 10:1 bis 1:10, insbesondere 2,5:1 bis 1:2,5. Ein bevorzugtes Verhältnis liegt bei etwa 1:1,5.

Als gegebenenfalls mitzuverwendende organische Lösungsmittel kommen inerte Kohlenwasserstoffe, wie Paraffinfraktionen, aromatische Kohlenwasserstoffe, wie Benzol, Toluol oder Xylol, oder ein Aldehyd und/oder Alkohol, insbesondere aber das Hydroformylierungsprodukt des eingesetzten Olefins, in Betracht. Es können ferner hochsiedende Nebenprodukte der Hydroformylierung als Lösungsmittel verwendet werden. Die Mitverwendung eines Lösungsmittels kann z. B. zur Viskositätserniedrigung bei langkettigen Olefinen vorteilhaft sein.

Die Temperatur bei der Hydroformylierung liegt im Allgemeinen bei 100 bis 250 °C, insbesondere 145 bis 200 °C. Die Umsetzung wird vorzugsweise bei einem Druck im Bereich von 20 bis 400 bar, insbesondere 200 bis 300 bar, durchgeführt.

Das gegebenenfalls vorgewärmte Einsatzolefin, Synthesegas sowie gegebenenfalls Katalysator oder -vorläufer werden einem vertikalen, hochzylindrischen Reaktor zugeführt, der mindestens zwei Reaktionsräume aufweist. Erfindungsgemäß erstrecken sich die Reaktionsräume im Wesentlichen in Reaktorlängsrichtung und sind durch Auftrennung des Reaktorinnenraums mittels Einbauten, die entsprechend im Wesentlichen in Reaktorlängsrichtung angeordnet sind, gebildet. "Im Wesentlichen in Reaktorlängsrichtung" bedeutet, dass die Reaktionsräume ihre größte Raumausdehnung in Richtung der Reaktorlängsachse haben und die Länge eines Reaktionsraums mehr als 50 %, vorzugsweise mehr als 60 %, der Reaktorlänge beträgt. Die Einbauten bilden jeweils einen allseitig umschlossenen Reaktionsraum mit einer Zuführung für das Reaktionsgemisch an einem Ende und einer Abführung für das Reaktionsgemisch am entgegengesetzten Ende. Das Reaktionsgemisch wird somit zunächst einem ersten Reaktionsraum von unten zugeführt, es durchströmt denselben von unten nach oben und wird am oberen Ende desselben über geeignete Rückführungsmittel einem zweiten Reaktionsraum an dessen unteren Ende zugeführt. Aus diesem zweiten Reaktionsraum kann das Reaktionsgemisch nach außen abgeführt werden, es besteht aber auch die Möglichkeit, den Reaktor und das Verfahren so auszubilden, dass das Reaktionsgemisch vom oberen Ende des zweiten Reaktionsraums über Rückführungsmittel dem unterem Ende eines dritten Reaktionsraums zugeführt wird, gegebenenfalls in analoger Weise weitere Reaktionsräume durchströmt und schließlich vom oberen Teil des letzten Reaktionsraums nach außen geführt wird.

Die Einbauten sind bevorzugt als beidseitig geschlossene Zylinder ausgebildet, die jeweils an den beiden entgegengesetzten Enden Zu- bzw. Abführöffnungen aufweisen. Bezüglich der Anordnung der Einbauten im Reaktorquerschnitt gibt es grundsätzlich keine Einschränkungen; die Einbauten können nebeneinander, bevorzugt gleichmäßig über den Reaktorquerschnitt verteilt, besonders bevorzugt aber konzentrisch zueinander und zum Reaktormantel angeordnet sein.

In einer bevorzugten Ausführungsform bilden die Einbauten einen einzigen beidseitig geschlossenen Innenzylinder mit Zuführung am unteren Ende und Abführung am oberen Ende desselben. Das Reaktionsgemisch wird besonders bevorzugt zunächst dem Zwischenraum zwischen Reaktorinnenwand und Einbauten zugeführt, am oberen Ende des Zwischenraums über Rückführungsmittel einem zweiten Reaktionsraum an dessen unteren Ende zugeführt, am oberen Ende desselben gegebenenfalls in weitere Reaktionsräume von unten nach oben und schließlich nach außen abgeführt.

In dieser Ausführungsform dient der zweite Reaktionsraum somit als Nachreaktionszone, d. h. zur Vervollständigung des im ersten Reaktionsraum erzielten Olefinumsatzes ohne unerwünschte Rückvermischung mit dem Inhalt des ersten Reaktionsraums.

Gemäß einer möglichen Ausführungsvariante kann nicht umgesetztes Synthesegas vom Gasraum am oberen Ende eines oder mehrerer Reaktionsräume mit Ausnahme des letzten Reaktionsraums entnommen, z. B. abgesaugt, verdichtet und dem Reaktor von unten erneut zugeführt werden. Diese Maßnahme kann unabhängig von der Zahl der vorhandenen Reaktionsräume angewendet werden. Sind lediglich zwei Reaktionsräume vorhanden, so wird nicht umgesetztes Synthesegas z. B. aus dem oberen Bereich des Zwischenraums zwischen Reaktorinnenwand und Einbauten abgezogen und demselben Reaktionsraum in dessen unteren Bereich erneut zugeführt. Bei mehr als zwei Reaktionsräumen kann nicht umgesetztes Gas einem oder mehreren der Reaktionsräume jeweils in dessen oberem Teil abgezogen werden, nicht jedoch dem Reaktionsraum, der als letzter vom Reaktionsgemisch durchströmt wird. Die Rückführung von Synthesegas erhöht die Turbulenz im ersten Reaktionsraum und sorgt für einen innigen Kontakt der Gas- und Flüssigphase.

Die Verdichtung des rückgeführten, nicht umgesetzten Synthesegases kann vorteilhaft mittels einer Strahlpumpe erfolgen, wobei es besonders günstig ist, die Strahlpumpe mit dem Olefin und frisch zugeführtes Synthesegas umfassenden Eduktstrom zu betreiben. Die Strahlpumpe kann im unteren Bereich des Reaktors angeordnet sein, es ist jedoch auch möglich, dieselbe unterhalb des Reaktors, außerhalb desselben anzuordnen.

Gemäß einer weiteren Ausführungsvariante ist es möglich, die Strahlpumpe zusätzlich zum Eduktstrom mit teilumgesetztem Reaktionsgemisch, das z. B. mittels einer Kreislaufpumpe am unteren Ende des ersten Reaktionsraums aus dem Reaktor abgesaugt wird, zu betreiben. Besonders bevorzugt kann das abgesaugte Reaktionsgemisch vor der Zuführung zur Strahlpumpe zwecks Wärmeabführung zunächst über einen Wärmetauscher geführt werden.

Zwischen den einzelnen Reaktionsräumen im Inneren des Reaktors sind Rückführungsmittel vorgesehen, die jeweils das Reaktionsgemisch vom oberen Ende eines Reaktionsraums zum unteren Ende eines nächsten Reaktionsraums führen. Diese Rückführungsmittel sind besonders bevorzugt als Fallrohre oder Syphone ausgebildet. Die Fallrohre sind so ausgebildet, dass durch ihre obere Kante der Flüssigkeitsstand, d. h. die Flüssig-Gas-Phasengrenze, im entsprechenden Reaktionsraum konstant gehalten wird. Sollte der Flüssigkeitsstand unter die Oberkante des Fallrohrs sinken, wird aufgrund des sich aufbauenden Gasdrucks vermehrt nicht umgesetztes Synthesegas über das Fallrohr in den nächsten Reaktionsraum geleitet, bis der Flüssigkeitsstand wieder die Oberkante des Fallrohrs erreicht, und umgekehrt. Auf diese Weise wird eine Pegelsteuerung ohne aufwendige technische Maßnahmen erreicht.

Der hochzylindrische Reaktor weist bevorzugt einen Schlankheitsgrad, d. h. ein Verhältnis von Länge zu Durchmesser, 1/d von etwa 3:1 bis 30:1, insbesondere von etwa 5:1 bis 10:1 auf.

Die Einbauten im Reaktor sind bevorzugt in der Weise dimensioniert, dass das Verhältnis des Volumens des ersten Reaktionsraums zu dem des zweiten Reaktionsraums bzw. zweier weiterer aufeinanderfolgender Reaktionsräume 4:1 bis 1:4, bevorzugt 1,5:1 bis 1:1,5, besonders bevorzugt etwa 1:1 beträgt.

Die Dimensionierung der Einbauten in die Längsrichtung ist in der Weise ausgestaltet, dass die Höhe des zweiten Reaktionsraums (der weiteren Reaktionsräume) mehr als 50 %, insbesondere mehr als 60 %, der Höhe des Reaktors, besonders bevorzugt 80 bis 95 % der Höhe des Reaktors beträgt.

Bevorzugt kann eine zusätzliche Kaskadierung des letzten Reaktionsraums, z. B. des zweiten Reaktionsraums, mittels horizontaler diffusionshemmender Einrichtungen, z. B. Lochbleche, die vorzugsweise äquidistant zueinander angeordnet sind, erreicht werden. Die Anzahl der Lochbleche kann 1 bis 10, besonders bevorzugt 3 bis 5 betragen. Diese Maßnahme wirkt sich umsatzfördernd auf die Hydroformylierungsreaktion aus, so dass eine weitere Umsatzsteigerung bei gegebenem Reaktionsvolumen erreicht werden kann. Die horizontalen Lochbleche können vorteilhaft außerhalb des Reaktors in die die weiteren Reaktionsräume bildenden Einbauten montiert werden, so dass die Montagekosten deutlich reduziert werden. Sie können dabei sowohl lösbar, durch entsprechende Flanschverbindungen montiert, oder aber direkt mit den Einbauten verschweißt werden.

Der erfindungsgemäße Reaktor kann in seinem Inneren Mittel zur indirekten Kühlung der Reaktionsräume aufweisen. Derartige Mittel sind bevorzugt insbesondere im ersten, vom Reaktionsgemisch durchströmten Reaktionsraum vorgesehen. Sie können bevorzugt als Rohrschlangen, die von einem Kühlmedium durchströmt werden, ausgebildet sein, wobei die Rohrschlangen auf den Einbauten aufgebracht, z. B. aufgeschweißt, oder beabstandet dazu angeordnet sein können.

Die Erfindung wird im Folgenden anhand einer Zeichnung und von Ausführungsbeispielen näher verdeutlicht:

Es zeigen im Einzelnen:
- Figur 1: die schematische Darstellung einer bevorzugten Ausführungsform einer erfindungsgemäßen Vorrichtung im Längsschnitt,
- Figur 2: eine weitere bevorzugte Ausführungsform einer erfindungsgemäßen Vorrichtung im Längsschnitt mit Gasrückführung über eine Strahlpumpe,
- Figur 3: eine weitere Ausgestaltung der Vorrichtung aus Figur 2 mit zusätzlicher Rückführung eines Teils des Reaktionsgemisches über die Strahlpumpe,
- Figur 4: eine weitere bevorzugte Ausgestaltung im Längsschnitt mit auf den Einbauten aufgebrachten Rohrschlangen,
- Figur 5: eine Ausführungsvariante mit Rohrschlangen, die nicht auf den Einbauten aufgebracht sind,
- Figur 6: eine Ausführungsvariante mit drei Reaktionsräumen im Längsschnitt sowie im Querschnitt (Figur 6a) und
- Figur 7a: einen Querschnitt durch einen erfindungsgemäßen Reaktor mit nicht konzentrischen Einbauten.

In den Figuren werden gleiche Merkmale mit gleichen Bezugsziffern bezeichnet.

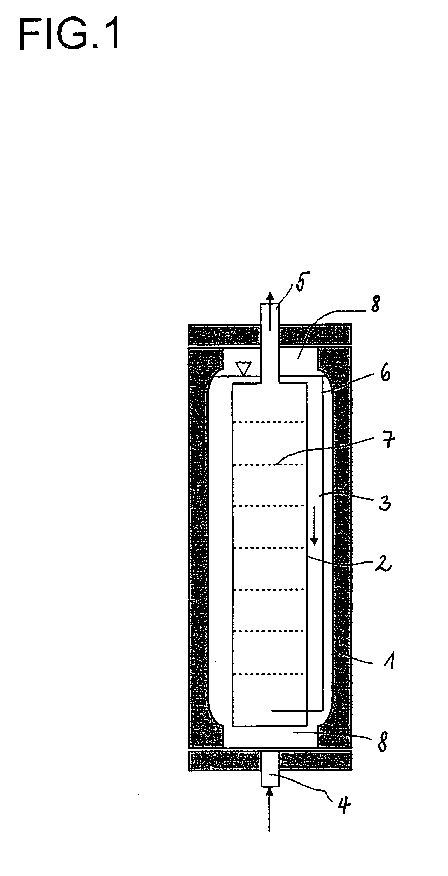

Fig. 1 zeigt beispielhaft einen erfindungsgemäßen Reaktor 1 im Längsschnitt mit jeweils einem Flaschenhals 8 an beiden Reaktorenden, mit Einbauten 2, die einen konzentrischen, beidseitig geschlossenen Innenzylinder bilden mit einer Zuführung 4 in den Zwischenraum 3 zwischen der Reaktorinnenwand und den Einbauten 2 und einer Abführung 5 aus dem durch die Einbauten 2 abgetrennten Reaktionsraum, mit Rückführungsmittel 6 aus dem oberen Teil des Zwischenraums zwischen Reaktorinnenwand und Einbauten 2 zum unteren Ende des durch die Einbauten 2 abgetrennten Reaktionsraums sowie mit horizontalen Lochblechen 7, wobei beispielhaft eine Anzahl von sieben Lochblechen dargestellt ist.

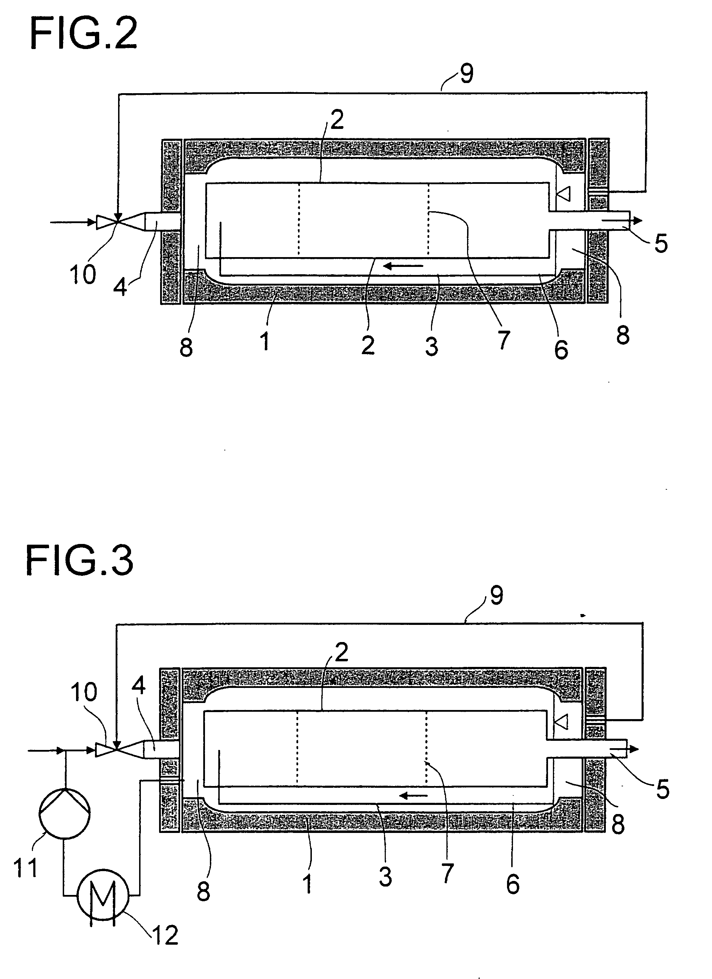

Fig. 2 zeigt eine weitere bevorzuge Ausführungsvariante im Längsschnitt, die zusätzlich eine Rückführleitung für Gas 9 aus dem oberen Bereich des Zwischenraums zwischen Reaktorinnenraum und Einbauten 2 aufweist, wobei die Rückführleitung 9 zu einer unterhalb des Reaktors angeordneten Strahlpumpe 10 führt, die durch das flüssige Eduktgemisch betrieben wird.

Fig. 3 stellt eine weitere bevorzugte Ausführungsform im Längsschnitt dar, wobei die unterhalb des Reaktors angeordnete Strahlpumpe 10 zusätzlich mit flüssigem Reaktionsgemisch betrieben wird, dass aus dem unteren Teil des Reaktors mittels einer Kreislaufpumpe 11 abgezogen und der Strahlpumpe zugeführt wird, und das flüssige Reaktionsgemisch zunächst über einen Wärmetauscher 12 gekühlt wird.

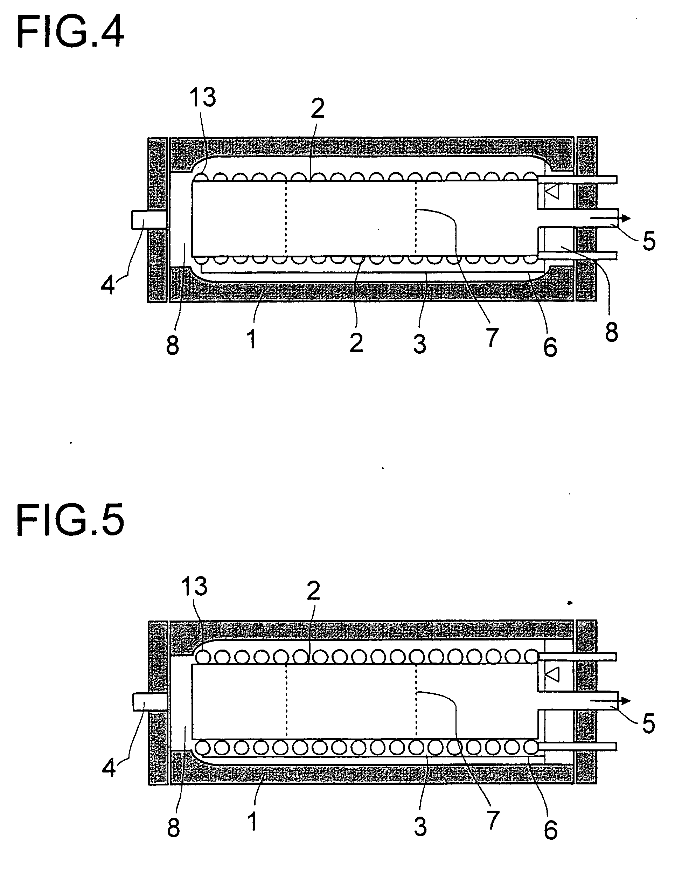

Die in Fig. 4 im Längsschnitt dargestellte Ausführungsvariante zeigt zusätzlich Rohrschlangen 13, die auf den Einbauten 2 aufgebracht sind und somit den Zwischenraum zwischen Reaktorinnenwand und Einbauten 2 wie auch den durch die Einbauten 2 abgetrennten Reaktorinnenraum kühlen.

Demgegenüber zeigt die Ausführungsvariante in Fig. 5 Rohrschlangen 13 die lediglich im Zwischenraum zwischen Reaktorinnenwand und Einbauten 2 angeordnet sind und somit überwiegend diesen Zwischenraum kühlen.

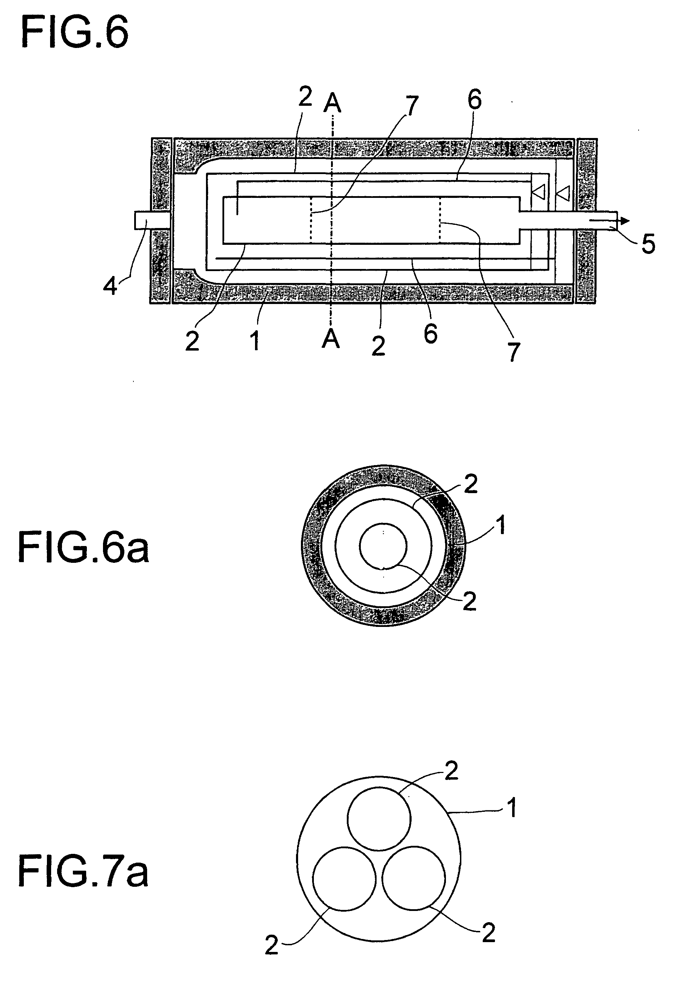

Fig. 6 zeigt ein Ausführungsbeispiel mit drei Reaktionsräumen, die durch konzentrische Anordnung von zwei zylindrischen Einbauten 2 innerhalb des Reaktors 1 gebildet werden. Der Querschnitt A/A durch den in Fig. 6 dargestellten Reaktor (Fig. 6a) verdeutlicht die konzentrische Anordnung der Einbauten 2.

Fig. 7a zeigt demgegenüber einen Querschnitt durch einen Reaktor mit drei im Reaktor 1 angeordneten nicht konzentrischen Einbauten 2.

Erfindungsgemäß wird somit ein Verfahren zur Verfügung gestellt, dass sich durch eine verbesserte Raumzeitausbeute, eine Erhöhung von Umsatz und Selektivität, auszeichnet. Die erfindungsgemäße Vorrichtung weist niedrigere Investitions- und Betriebskosten gegenüber bekannten Vorrichtungen aus.

Besonders vorteilhaft ist die Verbesserung des Stoffaustauschs zwischen flüssigem Reaktionsmedium und Gasphase infolge der erfindungsgemäßen Aufteilung des Reaktorquerschnittes in mehrere Zonen durch die im Wesentlichen im Reaktionslängsrichtung angeordneten Einbauten. Dadurch wird in den Zonen eine Vergrößerung der Querschnittsbelastung erreicht mit der Folge eines verbesserten Stoffaustausches. Dadurch können die bei bekannten Vorrichtungen sehr geringen Gasgehalte und Stoffaustauschflächen ohne den Einsatz von rotierenden Teilen, beispielsweise von Rührern, auf ein mehrfaches gesteigert werden. Die Nachteile, die mit dem Einsatz rotierender Teile, insbesondere bei Hochdruckapparaten verbunden sind, können so vermieden werden.

### Beispiel

Die Erfindung wird durch die folgende mathematische Simulation näher veranschaulicht. Die Simulation beruht auf einem kinetischen Modell der Hydroformylierung von Polyisobuten, das erstellt worden ist, indem eine Vielzahl experimenteller Messergebnisse mathematisch angepasst wurde. Als Zulauf wurde ein Gemisch von 80 Gew.-% eines leicht umsetzbaren, gering verzweigten Polyisobutens A₁ und 20 Gew.-% eines schwer umsetzbaren, stärker verzweigten Polyisobutens A₂ unterstellt. Es wurde ein 10%iger Überschuss an Synthesegas (molares Verhältnis CO/H₂ 1:1), eine Reaktionstemperatur von 130 °C und ein Druck von 280 bar zugrunde gelegt. Es wurde angenommen, dass die Reaktionsgeschwindigkeiten der Umsetzung von A₁ und A₂ zum jeweiligen Hydroformylierungsprodukt proportional den Molanteilen von A₁ bzw. A₂ sind. Eine Abhängigkeit von der CO- bzw. H₂-Konzentration wurde vernachlässigt. Es wurde unterstellt, dass sich durch die Reaktionen das Reaktionsvolumen nicht verändert. Der zugrunde gelegte Massenstrom an Olefinzulauf und Katalysatorlösung (wässrige Kobalt(II)-formiatlösung) betrug jeweils 6000 kg/h.

Im Fall I wurde ein Reaktor ohne Einbauten mit einem Volumen von 30 m³ und vollkommener Rückvermischung betrachtet. Im Fall II war der Reaktor in vier ideal durchmischte Teilstufen mit einem jeweiligen Volumen von 15 m³, 5 m³, 5 m³ und 5 m³ aufgeteilt. Die Eintrittskonzentrationen des Olefinzulaufs sowie die Katalysatormengen sind in beiden Reaktorkonfigurationen gleich.

Die erhaltenen Ergebnisse sind in der folgenden Tabelle zusammengefasst.

| Beispiel | Umsatz A₁ | Umsatz A₂ | Gesamtumsatz A₁+A₂ |
|---|---|---|---|
| I | 78,3 % | 26,5 % | 67,9 % |
| II | 91,3 % | 28,9 % | 78,8 % |

Ersichtlich sind bei der erfindungsgemäßen Kaskadierung des Reaktors bei gleichem Reaktionsvolumen sowohl die Einzelumsätze als auch der Gesamtumsatz höher.

## Patentansprüche

1. Kontinuierliches Verfahren zur Hydroformylierung von Olefinen mit wenigstens 6 Kohlenstoffatomen unter homogener Katalyse, bei dem man
a) einen vertikalen hochzylindrischen Reaktor (1) verwendet, dessen Innenraum mittels Einbauten (2) in wenigstens zwei Reaktionsräume unterteilt ist, die zumindest einen ersten und einen letzten Reaktionsraum umfassen und die sich im Wesentlichen in Reaktorlängsrichtung erstrecken und deren Länge mehr als 50 % der Reaktorlänge beträgt,
b) wenigstens ein Olefin zusammen mit Synthesegas am unteren Ende des ersten Reaktionsraums in den Reaktor einführt,
c) vom oberen Ende eines Reaktionsraumes ein teilumgesetztes Reaktionsgemisch zum unteren Ende eines nächsten Reaktionsraums führt; und
d) am oberen Ende des letzten Reaktionsraums das hydroformylierte Olefin entnimmt.

2. Verfahren nach Anspruch 1, bei dem man einen Reaktor (1) verwendet, dessen Innenraum mittels Einbauten (2) so unterteilt ist, dass der zweite und gegebenenfalls weitere Reaktionsräume im Wesentlichen konzentrisch zum Reaktormantel angeordnet sind.

3. Verfahren nach Anspruch 1 oder 2, bei dem man nicht umgesetztes Synthesegas vom Gasraum am oberen Ende eines oder mehrerer Reaktionsräume mit Ausnahme des letzten Reaktionsraums entnimmt und erneut in den Reaktor einführt.

4. Verfahren nach Anspruch 3, bei dem man das entnommene Synthesegas mittels einer Strahlpumpe (10) in den Reaktor einführt, die mit dem zugeführten Olefin und Synthesegas betrieben wird.

5. Verfahren nach Anspruch 4, bei dem die Strahlpumpe (10) zusätzlich mit teilumgesetztem Reaktionsgemisch betrieben wird, das am unteren Ende des ersten Reaktionsraums entnommen wird.

6. Vertikaler hochzylindrischer Reaktor (1) zur Durchführung des Verfahrens nach einem der Ansprüche 1 bis 5, mit Einbauten (2), mittels welcher der Innenraum des Reaktors in wenigstens zwei Reaktionsräume unterteilt ist, die sich im Wesentlichen in Reaktorlängsrichtung erstrecken und deren Länge mehr als 50 % der Reaktorlänge beträgt, und Rückführungsmitteln (6) in Form wenigstens eines Fallrohres vom oberen Ende eines Reaktionsraums zum unteren Ende eines nächsten Reaktionsraums.

7. Reaktor (1) nach Anspruch 6, wobei der zweite und gegebenenfalls weitere Reaktionsräume im Wesentlichen konzentrisch zum Reaktormantel angeordnet sind.

8. Reaktor (1) nach Anspruch 6 oder 8 mit einem Schlankheitsgrad 1/d von 3:1 bis 30:1.

9. Reaktor (1) nach einem der Ansprüche 6 bis 8, wobei der letzte Reaktionsraum durch horizontale, beabstandet zueinander angeordnete Lochbleche (7) kaskadiert ist.

## Claims

1. A continuous process for the hydroformylation of olefins having at least 6 carbon atoms in the presence of a homogeneous catalyst, wherein
a) a vertical tall cylindrical reactor (1) whose interior space is divided by means of internals (2) into at least two reaction chambers which comprise at least one first and at least one last reaction chamber and extend essentially in the longitudinal direction of the reactor and whose length is more than 50% of the length of the reactor is used,
b) at least one olefin is introduced into the reactor together with synthesis gas at the lower end of the first reaction chamber,
c) a partially reacted reaction mixture is conveyed from the upper end of a reaction chamber to the lower end of a next reaction chamber; and
d) the hydroformylated olefin is taken off at the upper end of the last reaction chamber.

2. A process as claimed in claim 1, wherein a reactor (1) whose interior space is divided by means of internals (2) so that the second and any further reaction chambers are arranged essentially concentrically to the outer wall of the reactor is used.

3. A process as claimed in claim 1 or 2, wherein unreacted synthesis gas is taken from the gas space at the upper end of one or more reaction chambers with the exception of the last reaction chamber and is fed back into the reactor.

4. A process as claimed in claim 3, wherein the synthesis gas which has been taken off is fed back into the reactor by means of a jet pump (10) which is operated by means of the olefin and synthesis gas fed in.

5. A process as claimed in claim 4, wherein the jet pump (10) is additionally operated by means of a partially reacted reaction mixture which is taken off at the lower end of the first reaction chamber.

6. A vertical tall cylindrical reactor (1) for carrying out a process as claimed in any of claims 1 to 5 which is provided with internals (2) by means of which the interior space of the reactor is divided into at least two reaction chambers which extend essentially in the longitudinal direction of the reactor and whose length is more than 50% of the length of the reactor and means (6) in the form of at least one downpipe for the recirculation of fluid from the upper end of a reaction chamber to the lower end of a next reaction chamber.

7. A reactor (1) as claimed in claim 6, wherein the second and any further reaction chambers are arranged essentially concentrically to the outer wall of the reactor.

8. A reactor (1) as claimed in claim 6 or 8 having an aspect ratio l/d of from 3:1 to 30:1.

9. A reactor (1) as claimed in any of claims 6 to 8, wherein the last reaction chamber is cascaded by means of horizontal perforated plates (7) located at a distance from one another.

## Revendications

1. Procédé continu d'hydroformylation d'oléfines comportant au moins 6 atomes de carbone, sous catalyse homogène, dans lequel
a) on utilise un haut réacteur cylindrique vertical (1) dont le volume interne est subdivisé au moyen d'éléments encastrés (2) en au moins deux chambres réactionnelles qui comprennent au moins une première et une dernière chambre réactionnelle et qui s'étendent essentiellement dans la direction longitudinale du réacteur et dont la longueur est supérieure à 50 % de la longueur du réacteur,
b) on introduit dans le réacteur au moins une oléfine conjointement à du gaz de synthèse à l'extrémité inférieure de la première chambre réactionnelle,
c) on conduit depuis l'extrémité supérieure d'une chambre réactionnelle un mélange réactionnel ayant partiellement réagi vers l'extrémité inférieure d'une chambre réactionnelle suivante, et
d) on prélève à l'extrémité supérieure de la dernière chambre réactionnelle l'oléfine hydroformylée.

2. Procédé suivant la revendication 1, dans lequel on utilise un réacteur (1) dont le volume interne est subdivisé au moyen d'éléments encastrés (2) de façon que la deuxième chambre réactionnelle et éventuellement d'autres soient agencées sensiblement concentriquement à l'enveloppe du réacteur.

3. Procédé suivant la revendication 1 ou 2, dans lequel on prélève du gaz de synthèse qui n'a pas réagi à partir du volume gazeux à l'extrémité supérieure d'une ou de plusieurs chambres réactionnelles, à l'exception de la dernière chambre réactionnelle, et on l'introduit à nouveau dans le réacteur.

4. Procédé suivant la revendication 3, dans lequel on introduit dans le réacteur le gaz de synthèse prélevé au moyen d'une pompe à jet (10) qui est mise en service avec l'oléfine amenée et le gaz de synthèse.

5. Procédé suivant la revendication 4, dans lequel la pompe à jet (10) est en supplément mise en service avec un mélange réactionnel qui a partiellement réagi et qui est prélevé à l'extrémité inférieure de la première chambre réactionnelle.

6. Haut réacteur cylindrique vertical (1) pour la mise en oeuvre du procédé suivant l'une des revendications 1 à 5, comprenant des éléments encastrés (2), au moyen desquels le volume interne du réacteur est subdivisé en au moins deux chambres réactionnelles qui s'étendent sensiblement dans la direction longitudinale du réacteur et dont la longueur est supérieure à 50 % de la longueur du réacteur, et des moyens de recyclage (6) sous la forme d'au moins un tube de descente de l'extrémité supérieure d'une chambre réactionnelle à l'extrémité inférieure d'une chambre réactionnelle suivante.

7. Réacteur (1) suivant la revendication 6, dans lequel la deuxième chambre réactionnelle et éventuellement d'autres sont agencées sensiblement de manière concentrique à l'enveloppe du réacteur.

8. Réacteur (1) suivant la revendication 6 ou 7, présentant un degré d'allongement l/d de 3/1 à 30/1.

9. Réacteur (1) suivant l'une des revendications 6 à 8, dans lequel la dernière chambre réactionnelle est réalisée en forme de cascade par des plaques perforées horizontales (7), disposées à distance l'une de l'autre.
